# DEMANDE DE BREVET EUROPEEN

(11) **EP 3 009 156 A1**
(43) Date de publication de la demande: **20.04.2016**
(21) Numéro de dépôt: 14189391.7
(22) Date de dépôt: 17.10.2014
(51) Int. Cl.: A61M 5/14, A61M 1/16

(54) **Poignée d'un dispositif médical**

(71) Demandeur: DEBIOTECH S.A., 1004 Lausanne (CH)
(72) Inventeur: Krummenacher, Renaud, 1004 Lausanne (CH); Neftel, Frédéric, 1005 Lausanne (CH)
(74) Mandataire: Weihs, Bruno Konrad

(57) **Abrégé**

La poignée (12) d'un dispositif médicale divulgué par le présent document a une double fonction, la préhension du dispositif médical et le maintien d'un élément d'un système médicale contre ledit dispositif médical, de préférence un filtre de dialyse (4).

## Description

### Domaine de l'invention

L'invention concerne une poignée d'un dispositif médical qui permet d'appréhender le dispositif médical mais qui est également utilisé en tant que support pour un élément distinct du dispositif médical, par exemple un filtre.

### Etat de la technique

Certains dispositifs médicaux sont pourvus d'une poignée. Cette poignée n'est qu'un élément permettant de transporter ou manipuler le dispositif médical, autrement dit elle n'a pas d'autre fonction que la fonction classique d'une poignée. Or cet élément pratique a un certain coût pour une fonction relativement peu fondamentale.

D'un autre côté, certains systèmes tel que les systèmes de dialyse comprennent des éléments fonctionnels pour le traitement, mais ces éléments fonctionnels peuvent être des éléments distincts de l'appareil « principal ». Il s'agit par exemple du filtre d'un système d'hémodialyse qui est maintenu durant le traitement sur l'appareil dans une certaine position (verticale de préférence, pour permettre l'évacuation des bulles). Ainsi durant le traitement ces éléments sont maintenu ensembles. Ces systèmes médicaux sont donc pourvus de support n'ayant que la fonction de maintenir ledit élément contre l'appareil « principal ».

Autrement, chaque élément n'a qu'une fonction unique.

### Description générale de l'invention

La présente invention a pour but principal de palier les inconvénients des systèmes connus en proposant de fusionner la fonction de poignée et de support d'un élément. Une synergie est ainsi créée afin de minimiser le nombre de pièces dans un objectif économique et/ou d'optimisation des fonctions, de l'encombrement et/ou de rationaliser l'utilisation du dispositif.

Un système médical peut comprendre des dispositifs jetables et des dispositifs réutilisables (avec d'autres dispositifs jetables). De manière générale, lorsqu'un dispositif médical est mouillé par un fluide qui peut être le sang du patient, une solution médicamenteuse ou autre, ce dispositif est généralement jeté suite à son unique utilisation.

En particulier, dans le domaine de l'hémodialyse, le système médical comprend au moins un dispositif permanent qui comprend généralement les éléments les plus sophistiqués ou onéreux (par exemple : l'électronique, les capteurs, les actuateurs,...) et au moins un dispositif jetable. Il peut s'agir notamment de la distribution fluidique (cassette, tube, sac, poche de chauffage,...), du filtre et dans certains cas d'un dispositif sorbent qui permet de filtrer le dialysat utilisé afin de le recycler.

Lors de l'utilisation de tels systèmes médicaux, certains éléments du dispositif jetable peuvent être maintenus par le dispositif permanent. Généralement le filtre est maintenu par l'appareil principal (dispositif permanent) durant le traitement. En guise d'information, un filtre est un élément jetable dans lequel d'un côté le sang du patient circule et de l'autre un fluide (dialysat) dans le but d'effectuer les échanges nécessaires (urée, eau,...) entre ces deux fluides comme l'effectuerait un rein.

Dans le cadre de l'invention, le support (utilisé par exemple pour le filtre) sert également de poignée à un dispositif. L'intérêt est majeur, le filtre et son support forment ainsi un élément facilement appréhendable par un utilisateur, transportable et/ou manipulable en un seul élément tout en assurant la position fonctionnelle du filtre (angle).

Dans un mode de réalisation, le support peut servir également de poignée lorsque le filtre n'est pas agencé sur ledit support.

Dans un mode de réalisation, le filtre est maintenu sur un dispositif jetable. Ainsi, à la fin du traitement l'ensemble filtre et dispositif jetable est jeté sans avoir à les séparer. Cela permet de faciliter la manipulation de l'ensemble. Le dispositif jetable peut être le sorbent ou un système de distribution fluidique.

Dans un autre mode de réalisation, le filtre est maintenu sur un dispositif permanent, tout en permettant la préhension à travers ladite poignée du dispositif permanent.

Un des problèmes relatif à une poignée est qu'elle peut servir à déplacer un dispositif (permanent ou jetable) ayant un certain poids (500g ou plus). Un filtre (de forme plutôt tubulaire et/ou longitudinale) représente en lui-même une poignée idéale départ sa forme. Toutefois, si l'on se sert du filtre comme poignée, il faut s'assurer que le filtre est bien relié au dispositif de façon sécurisée pour de tels poids et que ce dernier ne risque pas d'être endommagé par une telle utilisation. L'originalité de l'invention est de se servir de la poignée comme support pour le filtre (avec la possibilité d'une fixation, par exemple au moyen d'un clipsage), tout en assurant la préhension du filtre à l'aide de la main au travers de la poignée elle-même. Ainsi c'est la poignée et non le filtre qui est soumis aux principales forces (tout en se servant de la forme cylindrique du filtre pour constituer la forme définitive de la poignée).

### Liste des figures

L'invention sera mieux comprise ci-après au moyen de quelques exemples illustrés.

Il va de soi que l'invention ne se limite pas à ces modes de réalisation.
Figure 1 schématise un exemple de système hémodialyse.
Figure 2 montre le dispositif comprenant une poignée telle que décrite par la présente invention.
Figure 3 illustre l'agencement du filtre sur la poignée.
Figure 4 illustre l'appréhension de l'ensemble par la main de l'utilisateur.
Figure 5 représente un système d'hémodialyse complet.

### Références numériques utilisées dans les figures

- 1: système d'hémodialyse
- 2: patient
- 3: appareil principal
- 4: filtre
- 5: sorbent
- 6: circuit du sang
- 7: circuit du dialysat
- 10: dispositif comprenant une poignée tel que décrite par l'invention
- 11: support du filtre
- 12: élément de préhension
- 13, 14: éléments de liaison mécanique
- 15: main de l'utilisateur

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées sans s'écarter de la portée ou l'esprit de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés dans le présent document dans un sens large et signifie de façon générale « inclut, mais sans s'y limiter.

Le terme "ou" est généralement employé dans un sens large comprenant "et/ou" à moins que le contexte indique clairement le contraire.

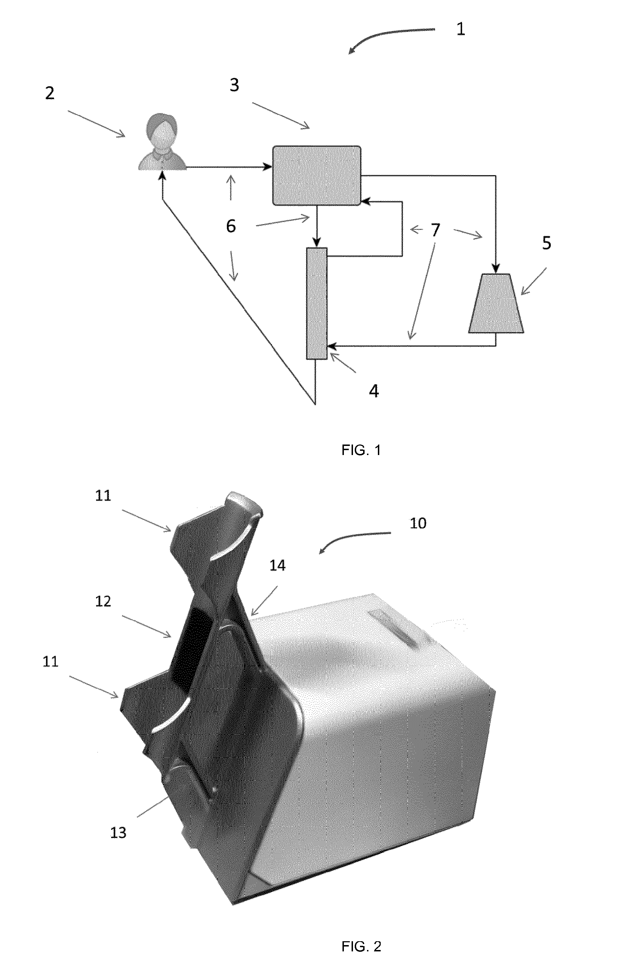

La figure 1 schématise brièvement un exemple de système d'hémodialyse (1). Le système comprend deux circuits distincts (6, 7), le premier est le sang et le deuxième le dialysat. Le sang est prélevé sur le patient grâce à une pompe à sang agencée dans l'appareil principale (3). Le sang va ensuite passer dans le filtre (4) afin d'être purifié puis il retourne dans le patient (2). Le circuit du dialysat (7) peut être différent en fonction des systèmes. Il peut comprendre un sac de dialysat frais qui va passer dans le filtre (4). Ensuite, ce dialysat utilisé peut être stocké dans un sac spécifique. Dans notre exemple, un dispositif de type sorbent (5) est agencé entre l'appareil principal (3) et le filtre (4). Dans d'autre système le sorbent peut être agencé ailleurs. Ainsi grâce au sorbent le dialysat utilisé peut être rincé de ses impuretés afin d'être réutilisé pendant le traitement (éventuellement par reconstitution du liquide avec un liquide contenant des solutés concentrés nécessaires à la reconstitution du dialysat à partir, notamment, d'eau).

Normalement tout ce qui a été mouillé par le sang ou le dialysat est à usage unique. Pour éviter de jeter l'appareil principal (3), le système utilise une cassette (non représenté) qui sert d'interface entre l'appareil principal et le fluide. De cette manière juste la cassette est jetée. La cassette, le filtre (4) et le sorbent (5) sont en communication fluidique à travers le circuit du dialysat (7) et sont donc, en général, destinés également à un usage unique.

Le filtre se présente comme un élément comprenant un corps principal longitudinal et deux extrémités opposés. A ces extrémités ou proche d'elle, des tubes permettent une connexion fluidique au circuit de sang et de dialysat.

La figure 2 illustre un mode de réalisation préférée, le dispositif (10) comprend une poignée solidement fixé au dispositif via au moins un moyen de liaison mécanique (13, 14). La poignée peut comprendre un élément de préhension (12) qui peut permettre à la main de l'utilisateur de passer entre la poignée et le dispositif (10). Grâce à cet élément de préhension, la poignée peut être utilisée sans le filtre mais elle permet également améliorer sensiblement la résistance mécanique de l'ensemble. La poignée comprend en outre éventuellement des supports pour maintenir de façon réversible le filtre sur l'élément de préhension.

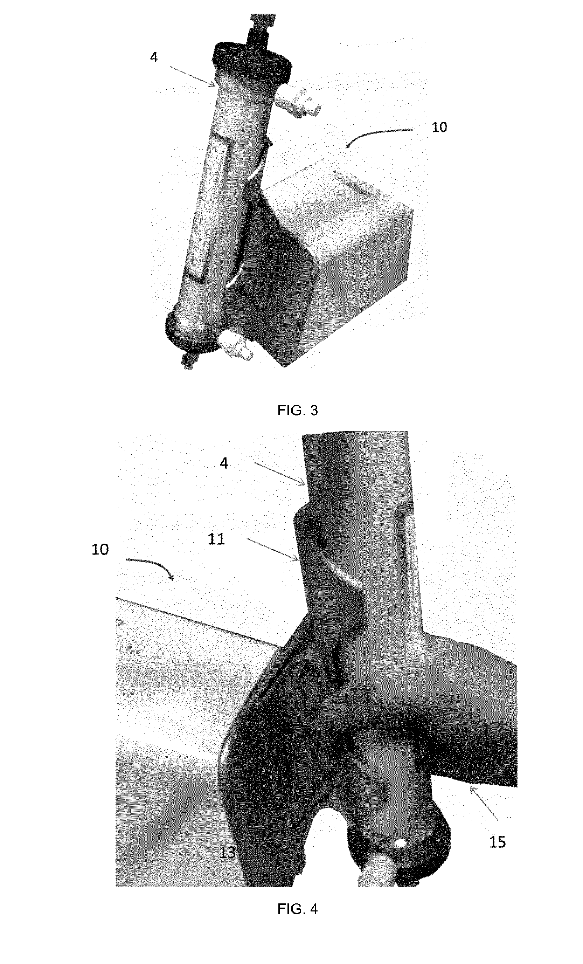

La figure 3 expose l'agencement du filtre (4) sur le dispositif (10). Dans ce mode de réalisation, l'élément de préhension (12) s'étend longitudinalement contre le corps du filtre (4). Le support peut comprendre un ou deux éléments agencés substantiellement aux deux extrémités de l'élément de préhension et ou proche des extrémités du filtre.

La figure 4 illustre l'appréhension de la poignée par la main d'un utilisateur (15). Ainsi, le filtre (4) et l'élément de préhension (12) forment un ensemble adapté pour être appréhendé facilement par la main d'un utilisateur alors que l'élément de préhension et le filtre sont des éléments totalement distincts. L'existence de cet élément de préhension (12) permet d'éviter de transférer l'essentiel des contraintes mécanique sur le corps du filtre (4) et ne nécessite donc pas une fixation nécessairement sécurisée du filtre sur la poignée. Autrement dit, même sur le dispositif est lourd, l'élément de préhension assure une résistance mécanique de sorte que le filtre ne subisse pas trop de contrainte qui risquerait de l'endommager ou de sorte que l'ensemble ne nécessite pas de fixation sécurisée entre le filtre et la poignée elle-même.

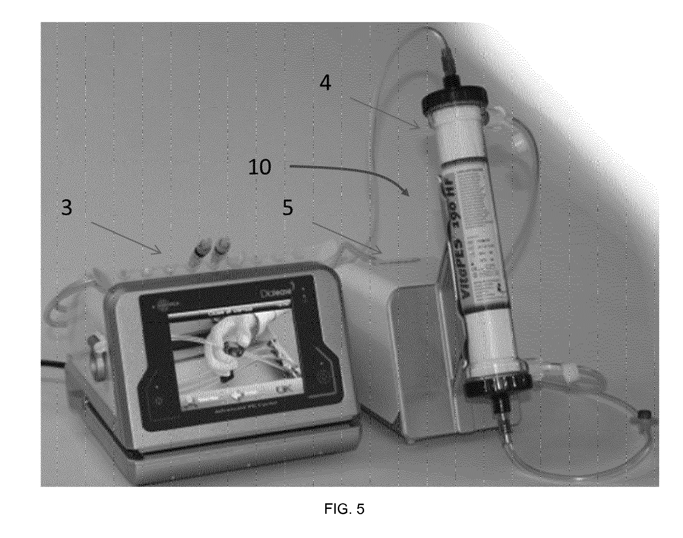

Dans un mode de réalisation divulgué par la figure 5, le système médical complet comprend un appareil principal (3), un filtre (4) maintenue sur le dispositif (10) comprenant la poignée. Dans ce mode de réalisation, le dispositif est un dispositif de sorbent (5).

Dans un mode de réalisation possible, l'élément de préhension (avec ou sans le filtre) peut être désolidarisé de son dispositif de sorte que il puisse être réutilisé avec un autre dispositif.

Dans un autre mode de réalisation, un ensemble constitué d'au moins deux éléments distincts forme une poignée d'un dispositif médiale. Cet ensemble comprend un premier élément et un deuxième élément distinct du dispositif médical et du premier élément. Cet ensemble est adapté pour être appréhendé par l'utilisateur au moyen d'une main. Le deuxième élément peut être de forme cylindrique (et/ou longitudinale) et maintenu de manière réversible au premier élément. Ainsi le deuxième élément est considéré comme un élément ergonomique pour la préhension tandis que le premier élément est considéré comme un élément de levage du dispositif. L'ensemble étant adapté pour être appréhendé ensemble par une main qui peut se saisir de l'ensemble pour le lever.

Le dispositif (10) peut être l'appareil principal ou un élément de cet appareil principal. Dans un autre mode de réalisation, le dispositif est un dispositif jetable qui peut comprendre un sorbent (5). Aussi, le filtre et le dispositif peuvent être en communication fluidique via des tubes ou directement à travers les éléments de liaison mécanique (13, 14).

## Revendications

1. Poignée pour un dispositif médical, ladite poignée comprenant :
• Un élément de préhension situé à une distance du dispositif médical permettant le passage des doigts
• Un élément de liaison mécanique permettant de relier solidement l'élément de préhension au dispositif médical
• Un support adapté pour recevoir et maintenir solidement un filtre sur l'élément de préhension
dans laquelle le filtre et l'élément de préhension sont des éléments distincts qui forment un ensemble poignée adapté pour être appréhendé par la main d'un utilisateur.

2. Poignée selon la revendication 1, dans laquelle l'élément de préhension s'étend longitudinalement le long du filtre.

3. Poignée selon l'une des précédentes revendications, dans laquelle le support comprend au moins un élément de fixation du filtre agencé sur l'élément de préhension.

4. Poignée selon l'une des précédentes revendications 1 à 2, dans laquelle le support comprend deux éléments de fixation du filtre agencés substantiellement aux deux extrémités de l'élément de préhension.

5. Poignée selon l'une des précédentes revendications, dans laquelle l'élément de préhension peut être détaché du dispositif médical.

6. Poignée selon l'une des précédentes revendications, dans laquelle l'élément de préhension avec le filtre peuvent être détachés ensemble du dispositif médical pour être placés sur un nouveau dispositif médical.

7. Dispositif médical comprenant une poignée décrite selon l'une des précédentes revendications, dans laquelle le dispositif médical est jetable ainsi que le filtre suite à une unique utilisation ou un nombre limité d'utilisations, de manière à ce que l'ensemble dispositif médical et filtre puisse être jeté facilement.

8. Dispositif selon la revendication précédente, dans lequel le dispositif est relié fluidiquement au filtre.

9. Dispositif selon l'une des précédentes revendications 7 à 8, dans lequel ledit dispositif médical est/ou comprend un sorbent pour fluide de dialyse.

10. Dispositif selon l'une des précédentes revendications 8 à 9, dans lequel le dispositif médical est un élément d'un système de dialyse péritonéale.

11. Dispositif selon l'une des précédentes revendications 8 à 9, dans lequel le dispositif médical est un élément d'un système d'hémodialyse.
